# EUROPÄISCHE PATENTANMELDUNG

(11) **EP 2 057 986 A1**
(43) Veröffentlichungstag der Anmeldung: **13.05.2009**
(21) Anmeldenummer: 07120358.2
(22) Anmeldetag: 09.11.2007
(51) Int. Cl.: A61K 9/48

(54) **Weichkapselhüllen auf Basis von Polyvinylalkohol-Polyethylenglykol-Pfropfcopolymeren enthaltend natürliche Polysaccharide**

(71) Anmelder: BASF SE, 67056 Ludwigshafen (DE)
(72) Erfinder: Kolter, Karl Dr., 67117 Limburgerhof (DE); Meyer-Böhm, Kathrin Dr., 90537 Feucht (DE)

(57) **Zusammenfassung**

Weichkapselhüllen, erhältlich aus Filmen enthaltend eine Polymerkomponente, wobei die Polymerkomponente aus einem Polyvinylalkohol-Polyether-Pfropfpolymeren oder einer Mischung aus Polyvinylalkohol-Polyether-Pfropfpolymeren und Polyvinylalkohol besteht, und ein natürliches Polysaccharid als Prozessierhilfsmittel.

## Beschreibung

Die vorliegende Erfindung betrifft die Verwendung von natürlichen Polysacchariden als Prozessierhilfsmittel für die verbesserte Herstellung von Weichkapselhüllen auf Basis von Polyvinylester-Polyethylenglykol-Pfropfcopolymeren.

Weichkapseln zeichnen sich dadurch aus, dass die Herstellung der Hülle und das Befüllen in einem Schritt nahezu simultan erfolgen. In der Regel besteht die Hülle solcher Weichkapseln hauptsächlich aus Gelatine, weshalb die Kapseln auch häufig als Weichgelatinekapseln bezeichnet werden. Da Gelatine an sich ein sprödes, wenig flexibles Material ist, muß es entsprechend weichgemacht werden, das heißt es müssen Weichmacher zugesetzt werden. Solche Weichmacher sind niedermolekulare Verbindungen, in der Regel Flüssigkeiten, wie z.B. Glycerin, Propylenglykol, Polyethylenglykol 400. Darüber hinaus enthalten solche Kapseln oft noch Farbstoffe, Opakisierungsmittel und Konservierungsstoffe.

Gelatine wird zwar häufig eingesetzt, jedoch weist sie zahlreiche Nachteile auf. So ist Gelatine ein Material tierischen Ursprungs und damit nicht kosher. Außerdem bleibt immer ein geringes Restrisiko von BSE, da zu ihrer Herstellung bevorzugt Gelatine von Rindern verwendet wird. Die Gewinnung einer geeigneten Gelatine ist sehr aufwendig und erfordert eine strenge Überwachung des Prozesses. Trotzdem sind die Chargenunterschiede aufgrund des tierischen Ursprungs, der einer gewissen Variabilität unterliegt, groß. Gelatine ist mikrobiell sehr anfällig, da sie einen guten Nährboden für Mikroorganismen darstellt. Bei der Herstellung, wie auch der Verwendung von solchen Verpackungsmaterialien müssen deshalb entsprechende Maßnahmen ergriffen werden. Häufig ist der Einsatz von Konservierungsmitteln unerläßlich.

Die bei der Herstellung von Gelatinekapseln unbedingt erforderlichen Weichmacher treten häufig von der Hülle in das Füllgut über und führen dort zu Veränderungen. Die Hülle verarmt an Weichmachern und wird im Laufe der Lagerung spröde und mechanisch instabil. Darüber hinaus besitzt eine Weichgelatinekapsel einen relativ hohen Wassergehalt in der Hülle, der ebenfalls eine weichmachende Wirkung hat. Werden solche Kapseln bei reiner Luftfeuchte gelagert, so verdunstet Wasser aus der Hülle, wodurch die Kapsel ebenfalls versprödet. Gleiches passiert auch, wenn sehr hygroskopische Güter verkapselt werden. Besonders hygroskopische oder hydrolyseempfindliche Stoffe können überhaupt nicht verkapselt werden.

Die Lösungsgeschwindigkeit von Gelatine ist verhältnismäßig langsam. Für schnelle Wirkstofffreisetzungen wäre eine höhere Auflösungsgeschwindigkeit in Magen- bzw.

Darmsaft wünschenswert.

Zahlreiche Stoffe führen mit Gelatine zu Interaktionen wie z.B. Aldehyde, Polyphenole, reduzierende Zucker, mehrwertige Kationen, Elektrolyte, kationische oder anionische Polymere etc., wobei häufig Vernetzung eintritt und die Kapsel nicht mehr oder nur noch ganz langsam zerfällt bzw. sich auflöst. Für ein Arzneimittel sind solche Veränderungen verheerend, da die Wirksamkeit nicht mehr gegeben ist. Auch viele Arzneistoffe führen mit Gelatine zu Interaktionen. Zum Teil bilden sich während der Lagerung Abbauprodukte von Arzneistoffen mit beispielsweise aldehydischer Struktur, die zu einer Vernetzung der Gelatine führen. Da Gelatine sowohl saure wie auch basische Gruppen aufweist, ist verständlich, daß Reaktionen mit anderen geladenen Molekülen leicht eintreten.

Gelatine kann enzymatisch gespalten werden. Verunreinigungen durch Enzyme bzw. von Bakterien abgesonderte Enzyme können die Eigenschaften von Gelatine dramatisch verändern.

Weichgelatinekapseln verkleben sehr leicht unter warmen und feuchten Bedingungen.

Die Haftung von Filmüberzügen auf Weichgelatinekapseln ist extrem schlecht. Häufig muß hierbei umständlich erst ein spezielles Subcoating aufgezogen werden.

Aufgrund dieser vielen Nachteile hat es nicht an Versuchen gefehlt, die Gelatine in Weichkapseln ganz oder teilweise zu ersetzen.

So werden beispielsweise in der US 6,340,473 Weichkapseln auf Basis von modifizierten Stärken und Carrageenanen beschrieben.

Polyvinylalkohol ist beispielsweise für diesen Zweck beschrieben. Polyvinylalkohol weist jedoch eine langsame Lösungsgeschwindigkeit auf, erfordert ebenfalls zusätzliche Weichmacher, die wiederum migrieren können und die, wie oben bereits beschrieben, die Eigenschaften des Füllguts verändern können, und kann außerdem in Folge innerer Kristallisation stark verspröden. Insbesondere bei niedriger Umgebungsfeuchte nimmt die Flexibilität im Laufe der Lagerung dramatisch ab.

DE-A2 2 363 853 beschreibt die Verwendung von teilverseiften Copolymerisaten von Vinylacetat und Polyethylenglykol zur Herstellung von Hartkapseln für Medikamente. Für die Verwendung der Copolymerisate zur Herstellung von Weichkapseln finden sich in dieser Schrift keine Hinweise.

An Hartkapseln werden jedoch ganz andere Anforderungen gestellt als an Weichkapseln. Hartkapseln benötigen eine hohe Festigkeit während bei Weichkapseln die Flexibilität im Vordergrund steht. Auch die Herstellungsverfahren sind völlig unterschiedlich. Bei Hartkapseln wird zunächst nur die Hülle in 2 separaten Teilen, einem Oberteil und Unterteil, mittels eines Tauchverfahrens hergestellt, während bei Weichkapseln die Herstellung der Hülle und die Füllung nahezu simultan verlaufen.

Bei den Hartkapseln werden nach der Herstellung von Oberteil und Unterteil diese locker ineinandergeschoben, so daß der pharmazeutische Hersteller die beiden Teile maschinell wieder trennen kann, sein Pulver einfüllen und die Kapsel verschließen kann. Bei näherer Betrachtung dieser Verarbeitung ist klar, daß die beiden Kapselteile mechanisch sehr stabil sein müssen, zumal die Füllmaschinen sehr schnell laufen und Formveränderungen den ganzen Prozeß lahmlegen würden.

Bei Weichkapseln muß die Hülle erstens absolut dicht sein, damit das Füllgut, das in der Regel flüssig ist, nicht austreten kann, und zweitens sehr flexibel sein, weil das Füllgut sonst durch Risse bzw. Mikrorisse austreten würde. Bei der Herstellung ist eine besonders hohe Flexibilität erforderlich, weil der Polymerfilm in Hohlbohrungen eingesaugt wird und damit stark verformt und gedehnt wird. Die Herstellung von Weichkapseln ist ein technologisch enorm anspruchsvoller Prozeß, von daher müssen die Polymereigenschaften und die Maschinen exakt angepaßt und eingestellt werden.

Die Anmeldung WO 97/35537 beschreibt ein spezielles Verfahren zur Herstellung von Weichkapseln unter Verwendung von verschiedenen Materialien, hauptsächlich Polyvinylalkohol. Vor der Verkapselung wird ein Lösungsmittel auf den Film aufgebracht, um ihn anzulösen, damit die Verklebung besser erfolgen kann. Dies ist allerdings nur bei entsprechend schwer zu verarbeitenden Filmen erforderlich.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

Gemäß der US-A 3,984,494 werden Polyvinylalkohol-Polyether-Pfropfpolymere für die Herstellung von Hartkapseln beschrieben.

In der EP-A 1136070 sind Weich kapseln aus Polyvinylalkohol-Polyether-Pfropfpolymeren beschrieben. Zur Herstellung der Kapselhüllen werden die Polymere zunächst in Wasser gelöst und dann zu Filmen ausgezogen. Allerdings lassen die so erhaltenen Filme hinsichtlich ihrer Festigkeit und Verarbeitungsfähigkeit noch Raum für Verbesserungen.

In der WO 2007/028758 ist ein verbessertes Verfahren zur Herstellung von Weichkapselhüllen durch Extrusion von wässrigen Lösungen beschrieben, wobei den Lösungen strukturverbessernde Hilfsstoffe zugesetzt werden können. Zu der Vielzahl an genannten Hilfsstoffen gehören unter anderem auch Polysaccharide.

Aufgabe der vorliegenden Erfindung war es, ein verbessertes Verfahren zur Herstellung von Weichkapselhüllen auf Basis von Polyvinylalkohol-Polyether-Pfropfcopolymeren zu finden.

Demgemäß wurde die Verwendung von natürlichen Polysacchariden als Prozessierhilfsmittel bei der Herstellung von Weichkapseln auf Basis von Polyvinylakohol-Polyether-Pfropfpolymeren sowie ein Verfahren zur Herstellung von Weichkapselhüllen auf Basis von Polyvinylakohol-Polyether-Pfropfpolymeren gefunden, welches dadurch gekennzeichnet ist, dass eine wässrige Lösung, enthaltend mindestens 45 Gew.-% einer Polymerkomponente, welche aus Polyvinylalkohol-Polyether- Pfropfpolymer oder einer Mischung des Polyvinylalkohol-Polyether- Pfropfpolymers mit Polyvinylalkohol besteht, und mindestens ein natürliches Polysaccharid als Prozessierhilfsmittel zu Filmen verarbeitet und verfestigt werden.

Geeignete Polyvinylalkohol-Polyether-Pfropfcopolymere sind in der WO 2007/028758 beschrieben auf die hinsichtlich der Offenbarung hiermit Bezug genommen wird.

Insbesondere lässt sich mit Hilfe des erfindungsgemäßen Verfahrens ein Pfropfpolymer verarbeiten, das durch Polymerisation von 15 Gew.-% Polyetheranteil und 85 Gew.-% Vinylacetat erhalten wurde. Das Molekulargewicht der Polyetherkomponente beträgt dabei bevorzugt 6000 Dalton. Der Verseifungsgrad beträgt bevorzugt 88-98 %.

Als weitere Polymer-Komponente können die Weichkapselhüllen Polyvinylalkohol enthalten. Bevorzugt werden Polyvinylalkohole mit mittleren Molekulargewichten M_{w} von 1000 bis 500000, bevorzugt 5000 bis 100000 und besonders bevorzugt 10000 bis 50000 eingesetzt. Das Gewichtsverhältnis von Polyvinylester-Polyether-Pfropfpolymeren zu Polyvinylalkoholen kann 100:0 bis 30:70, bevorzugt 100:0 bis 50:50, betragen.

Als Prozessierhilfsmittel werden erfindungsgemäß natürliche Polysaccharide eingesetzt. Diese Prozessierhilfsmittel bewirken eine verbesserte Verarbeitbarkeit der wasserhaltigen Filme durch Viskositätserhöhung.

### Als natürliche Polysaccharide kommen in Betracht:

Stärke, beispielsweise Maisstärke, Kartoffelstärke, Reisstärke, Weizenstärke, Tapiokastärke, Erbsenstärke, Agar, Alginate, Pektin, Guar, Aloe, Xanthan, Gellan, Wellan, Rhamsan, Dextran, Curdlan, Pullulan, Schroglucan, Chitosan, Tamarindenextrakt oder Bockshornkleextrakt. Weiterhin eignen sich Carrageenane, insbesondere Kappa-Carrageenan.

Bevorzugt sind Caraggenane und Stärken, wobei die Stärken besonders bevorzugt sind.

Die Mengen an natürlichen Polysacchariden können 0.05 bis 10, bevorzugt 0.1 bis 7 Gew.-% betragen.

Carrageenane werden bevorzugt in Mengen von 0.05 bis 0.5 Gew.-% eingesetzt, Stärken bevorzugt in Mengen von 2 bis 7 Gew.-%.

Zur Durchführung des erfindungsgemäßen Verfahrens wird zunächst eine Lösung der Pfropfpolymeren oder der Mischung des Pfropfpolymers mit dem Polyvinylakohol und gegebenenfalls weiterer Hilfsstoffe hergestellt, wobei der Feststoffgehalt der Lösung so gewählt wird, dass der Gehalt an Pfropfpolymeren oder an der Summe aus Pfropfpolymeren und Polyvinylalkohol mindestens 45 Gew.-% und bis zu 75 Gew.-%, bevorzugt 50 bis 70 Gew.-%, bezogen auf das Gesamtgewicht der wässrigen Lösung, beträgt.

Die Lösung wird vor der Filmbildung auf eine Temperatur von mindestens 70 °C und bis zu 120°C, bevorzugt 75 bis 100°C gebracht. Anschliessend wird die heiße Lösung zu einem Film geformt und abgekühlt.

Die Herstellung der Lösungen kann in jeder beliebigen dafür geeigneten Vorrichtung erfolgen, beispielsweise in temperaturregulierbaren Rührbehältern oder in Extrudern.

Die Verformung zu einem Film kann auf jede bekannte Methode wie beispielsweise Ausgießen und Rakeln oder Extrusion beispielsweise durch eine Schlitzdüse erfolgen.

Bevorzugt erfolgt die Filmbildung durch Extrusion. Ein geeignetes Verfahren ist in der WO 2007/028758 beschrieben, auf deren Offenbarung diesbezüglich ausdrücklich Bezug genommen wird.

Geeignete Filmdicken liegen zwischen 100 und 2000 µm, vorzugsweise zwischen 150 und 1000 µm.

Die erfindungsgemäß hergestellten als Weichkapselhüllen geeigneten Filme weisen insbesondere folgende Eigenschaften auf:
- Reißdehnung:: 50 - 600% in feuchtem Zustand 25 - 300% in getrocknetem Zustand (bei einer Gleichgewichtsfeuchte von 53% relativer Feuchte.)
- Zugfestigkeit:: 2 - 30N/mm2 in feuchtem Zustand 5 - 60N/mm2 in getrocknetem Zustand (bei einer Gleichgewichtsfeuchte von 53% relativer Feuchte)

Neben den Polyvinylalkohol-Polyether-Pfropfpolymeren und gegebenenfalls dem Polyvinylalkohol können den zur Herstellung der Weichkapselhüllen verwendeten Mischungen noch weitere Hilfsstoffe zugegeben werden.

So können 0,1 bis 1 Gew.-%, bezogen auf das Gesamtgewicht an Polymeren, an hochdisperser Kieselsäure, zugegeben werden.

Zur Erzielung einer Magensaftresistenz können in der Hülle 20 bis 80%, vorzugsweise 30 bis 70% eines magensaftresistenten Polymers enthalten sein.

Neben den genannten Komponenten können die erfindungsgemäßen Weichkapselhüllen noch weitere übliche Bestandteile enthalten. Dazu zählen Füllstoffe, Formtrennmittel, Rieselhilfsmittel, Stabilisatoren sowie wasserlösliche oder wasserunlösliche Farbstoffe, Aromen und Süßstoffe.

Farbstoffe sind z.B. Eisenoxide, Titandioxid, die in einer Menge von etwa 0.001 bis 10, vorzugsweise von 0.5 bis 3 Gew.% zugesetzt werden, Triphenylmethanfarbstoffe, Azofarbstoffe, Chinolinfarbstoffe, Indigofarbstoffe, Carotinoide, um die Kapseln einzufärben, Opakisierungsmittel wie Titandiodid oder Talkum, um die Lichtundurchlässigkeit zu erhöhen und um Farbstoffe einzusparen.

Aromen und Süßstoffe sind insbesondere dann von Bedeutung, wenn ein schlechter Geruch oder Geschmack überdeckt werden soll und die Kapsel zerbissen wird.

Konservierungsmittel sind in aller Regel nicht erforderlich.

Füllstoffe sind z.B. anorganische Füllstoffe wie Oxide von Magnesium, Aluminium, Silicium, Titan oder Calciumcarbonat. Der bevorzugte Konzentrationsbereich für die Füllstoffe ist etwa 1 bis 50 Gew.-%, besonders bevorzugt 2 bis 30 Gew.-% bezogen auf das Gesamtgewicht sämtlicher Komponenten.

Schmiermittel sind Stearate von Aluminium, Calcium, Magnesium und Zinn, sowie Magnesiumsilikat, Silikone und ähnliche. Der bevorzugte Konzentrationsbereich ist etwa 0.1 bis 5 Gew.-%, besonders bevorzugt etwa 0.1 bis 3 Gew.-% bezogen auf das Gesamtgewicht sämtlicher Komponenten.

Rieselhilfsmittel sind z.B. feinteilige bzw. feinstteilige Kieselsäuren, ggf. modifiziert. Der bevorzugte Konzentrationsbereich ist 0.05 bis 3 Gew.-%, besonders bevorzugt 0.1 bis 1 Gew.-% bezogen auf das Gesamtgewicht sämtlicher Komponenten.

Ein Sonderfall stellt die Einarbeitung von Wirkstoffen in die Hülle dar. Dies kann vorteilhaft sein, um inkompatible Wirkstoffe voneinander zu trennen. Der Wirkstoff mit der geringsten Dosierung sollte dann in die Hülle eingearbeitet werden.

Eine besonders bevorzugte Zusammensetzung enthält ein Pfropfpolymer und Polyvinylalkohol im Gewichtsverhältnis 6: 4. Ganz besonders bevorzugte Zusammensetzungen enthalten 0.25 Gew.-% , bezogen auf die Gesamtmenge an Pfropfpolymer plus Polyvinylalkohol, an hochdisperser Kieselsäure.

Die erfindungsgemäß erhaltenen Weichkapselhüllen bestehen aus 10 bis 99.95 %, vorzugsweise 20 bis 95 % Polymerisaten, 0.05 bis 10 Gew.-%, vorzugsweise 0.1 bis 7 Gew.-% natürlichen Polysacchariden und gegebenenfalls 0 bis 30 %, vorzugsweise 0,1 bis 30 % weiteren üblichen Bestandteilen.

Die Herstellung der befüllten Weichkapseln kann nach an sich für die Herstellung von Weichkapseln bekannten Verfahren erfolgen, beispielsweise nach dem Rotary-Die-Verfahren, dem Accogel-Verfahren, dem Norton-Verfahren, dem Tropf- oder Blasverfahren oder dem Colton-Upjohn-Verfahren. Diese Verfahren sind beschreiben in "W. Fahrig und U.Hofer, Die Kapsel, Wissenschaftliche Verlagsgesellschaft mbH, Suttgart, 1983".

Vor der Verarbeitung in der Verkapselungseinheit können die erfindungsgemäß erhaltenen als Weichkapselhüllen geeigneten Filme gewünschtenfalls mit Wasser oder mit Wasser mischbaren organischen Lösungsmittteln oder Gemischen aus Wasser und mit Wasser mischbaren Lösungsmitteln befeuchtet werden. Geeignete mit Wasser mischbare Lösungsmittel sind: Glycerin, 1,2-Propylenglykol, Polyethylenglykol mit Molekulargewichten zwischen 250 und 600. Dies empfiehlt sich insbesondere dann, wenn der zur Verkapselung verwendete Film nicht ausreichend weich und klebrig ist und somit die Verschweißung erschwert ist. Das oberflächliche Aufbringen dieser Stoffe erweicht den Film und verbessert die Verschweißbarkeit. Die Aufbringung kann durch Aufsprühen, Aufwalzen, Aufpinseln oder Aufrakeln erfolgen.
Die besonderen Vorzüge der beschriebenen Kapseln und des beschriebenen Verfahrens liegen darin, dass die Prozesszeiten für die Herstellung der Filme sehr kurz sind (nur wenige Minuten) und die Filme kaum Luftblasen enthalten. Durch die kurze Herstellungszeit kann die Geschwindigkeit der Filmherstellung an die Verkapselungsgeschwindigkeit adaptiert werden. Dadurch erfolgen Filmherstellung und Verkapselung in einem völlig kontinuierlichen Prozess. Dies ist unter Verwendung des Verfahrens von Filmziehung aus Polymerlösungen nicht möglich. Hier muß zunächst die Polymerlösung hergestellt werden, gerakelt und getrocknet werden.

Ferner ermöglicht das erfindungsgemäße Verfahren die einfache, individuelle Einstellung des Wassergehaltes nahezu ohne jegliche Einschränkung durch hohe Viskositäten. Dadurch sind sehr hohe Feststoffgehalte einstellbar. Demgegenüber kann das Rakeln von Filmen kann nur mit niedrigviskosen Lösungen erfolgen. Aufgrund der geringeren Menge an Wasser, die verdampft werden muß ergibt sich bei dem erfindungsgemäßen Verfahren eine wesentlich günstigere Energiebilanz.

Die Verschweissung kann ebenfalls mit hoher Geschwindigkeit sehr gleichmässig und reproduzierbar und ohne Risse oder Poren erfolgen, wodurch der Ausschuss an beschädigten Kapseln extrem gering ist. Ferner sind die Kapeln leicht zu trocknen, behalten hierbei ihre Form und Flexibilität und sind lagerstabil, da sie nicht wie z.B. Stärke das Phänomen der Retrogradation aufweisen. Die Auflösegeschwindigkeit der Hülle ist größer als bei den bekannten Kapseln und vor allem lösen sie sich auch in kalten wässrigen Medien.

Typische verpackte Materialien sind bevorzugt pharmazeutische Erzeugnisse, wie feste und flüssige Wirkstoffe, aber auch Vitamine, Carotinoide, Mineralstoffe, Spurenelemente, Nahrungsergänzungsstoffe, Gewürze sowie Süßstoffe. Weiterhin können die Kapseln für kosmetische Wirkstoffe ("personal care"), wie beispielsweise Haar- und Hautformulierungen, für Öle, Duftstoffe, Badezusätze oder Proteine verwendet werden. Weitere Anwendungen im Bereich "personal care" sowie weitere Anwendungen für wasserlösliche Verpackungen sind in der WO 99/40156 genannt.

Weitere verpackte Materialien können sein, z.B. Reinigungsmittel, wie Seifen, Waschmittel, Farb- und Bleichmittel, Agrarchemikalien wie Düngemittel (-kombinationen), Pflanzenschutzmittel wie Herbizide, Fungizide oder Pestizide und Saatgut.

Generell lassen sich Inhaltsstoffe verpacken, die geschützt werden sollen, bevor sie in eine wässrige Umgebung gebracht werden.

Die erfindungsgemäß erhaltenen Weichkapseln der erfindungsgemäßen Zusammensetzung lassen sich hervorragend unter Verwendung von wäßrigen Polymerlösungen oder Polymersuspensionen coaten. So kann durch Aufsprühen von Kollicoat MAE 30 DP (Methacrylsäure-Copolymer Typ C der USP) in einem Horizontaltrommelcoater ein stark auf der Oberfläche haftender magensaftresistenter Überzug aufgebracht werden, der zudem lagerungsstabil ist.

### Beispiele

Allgemeine Vorschrift zur Herstellung von Filmen für Weichkapselhüllen:
Die Verarbeitung erfolgte in einem Zweischneckenkneter ZSK 25 der Firma Coperion Werner & Pfleiderer mit 8 Zylindern, der einen Schneckendurchmesser von 25 mm bei einem L/D-Verhältnis von 34:1 aufwies. Die Zylindertemperatur betrug 90 bis 120 °C, die Schneckendrehzahl 120 bis 150 Upm.

Die Polymeren wurden in Zylinder 2 in den Extruder eingezogen und leicht erwärmt.
Über Injektordüsen wurden die entsprechenden Mengen Wasser und Weichmacher in Zylinder 4 zudosiert und die Mischung zur Auflösung der Polymeren auf 97°C erhitzt. Die Extrusion erfolgte bei einer Düsentemperatur von 93°C durch eine Schlitzdüse mit einer Breite von 100 mm und einer Höhe von 600 µm. Die austretenden Filme wurden auf 47° C abgekühlt . Die Prozesszeit für die Herstellung der Filme betrug 4min (Lösen des Polymers und Extrusion der Filme: 2 min, Abkühlung der Filme 2 min).
Die Filme enthielten keine Luftblasen.

Die konkrete Zusammensetzung der zur Herstellung der einzelnen Filme verwendeten Lösungen ist in der nachstehenden Tabelle aufgelistet.

Als Polymermischung für die Kapselhülle wurde eine Mischung aus einem Pfropfpolymer A erhalten aus 15 Gew.-% PEG 6000 und 85 Gew.- Vinylacetat mit einem verseifungsgrad von > 95 mol-% und einem K-Wert nach Fikentscher von 54 (1 gew.-%ig in N-Methylpyrrolidion)und einem Polyvinylalkohol B (M_{w} 37.000) im Gewichtsverhältnis A:B = 6:4 und 0.25 Gew.-%, bezogen auf die Gesamtmenge an A plus B, hochdisperse Kieselsäure verwendet.

**Wasser wurde ad 100 Gew.-% zugegeben.**

| Beispiel Nr. | Zusammensetzung Lösung | | |
|---|---|---|---|
| | Polymermischung | Polysaccharid | [Gew.-%] |
| | [Gew.-%] | | |
| 1 | 45 | - | 0 |
| (Vergleich 1) | | | |
| 2 | 45 | Maisstärke | 5 |
| 3 | 45 | Weizenstärke | 5 |
| 4 | 45 | Reisstärke | 5 |
| 5 | 45 | Kartoffelstärke | 5 |
| 6 | 50 | - | - |
| (Vergleich 2) | | | |
| 7 | 50 | Carrageenan | 0.1 |

**Mechanische Eigenschaften der Filme**

| Bsp.nr. | 1 (Vergleich 1) | 2 | 3 | 4 | 5 | 6 (Vergleich 2) | 7 |
|---|---|---|---|---|---|---|---|
| Reißfestigkeit [%] | 142 | 159 | 155 | 153 | 150 | 181 | 195 |
| E-Modul [N/mm2] | 25 | 39 | 36 | 40 | 38 | 27 | 33 |
| Zugspannung [N/mm2] | 5,29 | 6,54 | 6,11 | 6,34 | 6,20 | 5,41 | 6,68 |
| Aussehen des Films | klar | trüb | trüb | trüb | klar | klar | klar |

## Patentansprüche

1. Verfahren zur Herstellung von gelatinefreien Weichkapselhüllen basierend auf Polyvinylalkohol-Polyether-Pfropfpolymeren als Hüllpolymeren, **dadurch gekennzeichnet, dass** man eine wässrige Lösung, enthaltend mindestens 45 Gew.-%, bezogen auf das Gesamtgewicht der Lösung, einer Polymerkomponente, wobei die Polymerkomponente aus einem Polyvinylalkohol-Polyether-Pfropfpolymeren oder einer Mischung aus Polyvinylalkohol-Polyether-Pfropfpolymeren und Polyvinylalkohol besteht, sowie natürliche Polysaccharide als Prozessierhilfsmittel herstellt, die Lösung zu einem Film ausformt und den entstandenen Film durch Abkühlung verfestigt.

2. Verfahren nach Anspruch 1, **dadurch gekennzeichnet, dass** der wässrigen Lösung zusätzlich weitere Hilfsstoffe zugesetzt werden.

3. Verfahren nach Anspruch 1 oder 2, **dadurch gekennzeichnet, dass** das Gewichtsverhältnis von Polyvinylalkohol-Polyether-Pfropfpolymeren zu Polyvinylalkohol 100:1 bis 30:70 beträgt.

4. Verfahren nach einem der Ansprüche 1 bis 3, **dadurch gekennzeichnet, dass** als Prozessierhilfsmittel Stärken oder Carrageenane eingesetzt werden.

5. Verfahren nach einem der Ansprüche 1 bis 4, **dadurch gekennzeichnet, dass** man als Prozessierhilfsmittel Stärken einsetzt.

6. Verfahren nach einem der Ansprüche 1 bis 4, **dadurch gekennzeichnet, dass** die prozessierhilfsmittel in Mengen von 0.05 bis 10 Gew.-%, bezogen auf das Gesamtgewicht de wässrigen Lösung, eingesetzt werden.

7. Verfahren nach einem der Ansprüche 1 bis 5, **dadurch gekennzeichnet, dass** die Herstellung der wässrigen Lösung in einem Extruder erfolgt.

8. Verfahren nach einem der Ansprüche 1 bis 6, **dadurch gekennzeichnet, dass** die wässrige Lösung der Polymerkomponenten durch eine Schlitzdüse extrudiert wurde.

9. Verfahren nach einem der Ansprüche 1 bis 7, **dadurch gekennzeichnet, dass** die nach Abkühlung erhaltenen Filme vor Einbringen in die Verkapselungseinheit mit Wasser, einem mit Wasser mischbaren organischen Lösungsmittel oder einem Gemisch aus Wasser und einem mit Wasser mischbaren organischen Lösungsmittel angefeuchtet werden.

10. Verfahren nach einem der Ansprüche 1 bis 8, **dadurch gekennzeichnet, dass** die Filme zur Verfestigung auf eine Temperatur abgekühlt werden, die mindestens 10 °C unter der Extrusionstemperatur liegen.

11. Verwendung von natürlichen Polysacchariden als Prozessierhilfsmittel zur Herstellung von Weichkapselhüllen auf Basis einer Polymerkomponente, wobei die Polymerkomponente aus einem Polyvinylalkohol-Polyether-Pfropfpolymeren oder einer Mischung aus Polyvinylalkohol-Polyether-Pfropfpolymeren und Polyvinylalkohol besteht.

12. Weichkapselhüllen, erhältlich aus Filmen enthaltend eine Polymerkomponente, wobei die Polymerkomponente aus einem Polyvinylalkohol-Polyether-Pfropfpolymeren oder einer Mischung aus Polyvinylalkohol-Polyether-Pfropfpolymeren und Polyvinylalkohol besteht, und ein natürliches Polysaccharid als Prozessierhilfsmittel.

13. Weichkapselhüllen nach Anspruch 12, enthaltend 0.05 bis 10 gew.-% an natürlichen Polysacchariden.
